# EUROPEAN PATENT APPLICATION

(11) **EP 4 578 482 A1**
(43) Date of publication of application: **02.07.2025**
(21) Application number: 24222713.0
(22) Date of filing: 23.12.2024
(51) Int. Cl.: A61M 39/06

(54) **INSERTION TOOL FOR CATHETER DEVICES**

(30) Priority: 27.12.2023 US 202318397789
(71) Applicant: Biosense Webster (Israel) Ltd., 2066717 Yokneam (IL)
(72) Inventor: ABOYTES, Donald, Irvine, 92618 (US); BANANDO, Michael, Irvine, 92618 (US); KIM, Amie, Irvine, 92618 (US); SIU, Zachary, Irvine, 92618 (US); AGNEW V, William James, Irvine, 92618 (US)
(74) Representative: Carpmaels & Ransford LLP

(57) **Abstract**

The disclosed technology includes an insertion tool including a first body comprising a hemostatic valve configured to receive a flexible shaft and prevent fluid from flowing past the hemostatic valve. The insertion tool can include a second body coupled to the first body, with the second body including: a chamber, a guide tube smaller than the chamber, and a valve. The guide tube can be connected distal to the chamber and along a longitudinal axis of the second body to guide a flexible shaft into a sheath and the valve can be disposed at least partially in the guide tube. The valve can be biased to a closed position and upon insertion of a flexible shaft through the valve to prevent fluid from flowing past the valve.

## Description

### FIELD

The present invention relates generally to medical devices, and in particular medical probes with irrigation hubs, and further relates to, but not exclusively, medical probes configured with irrigation hubs configured to preserve sealing components during insertion of the medical probe into an anatomical region.

### BACKGROUND

Cardiac arrhythmias, such as atrial fibrillation (AF), occur when regions of cardiac tissue abnormally conduct electrical signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Some catheters comprise a basket design with a plurality of spines. An insertion tool may be utilized to prevent the introduction of air ingress by causing the basket to collapse for insertion into a sheath and ultimately into an anatomical region of a patient. Conventionally, such an insertion tool comprises a hemostatic valve at a first end and an open-ended guide tube to collapse the spines of the basket as it is inserted into the sheath. Unfortunately, interaction between the hemostatic valve and the spines of the basket catheter may damage the hemostatic valve. Further it is desirable to provide a valve to isolate or otherwise seal the guide tube of the insertion tool when the catheter is retracted from the sheath to prevent fluid egress. Accordingly, there is a need in the art for an insertion tool that limits interaction between the end effector and the hemostatic valve and provides an additional valve in the guide tube without obstructing the end effector during insertion into the sheath.

### SUMMARY

There is provided, in accordance with an example of the present invention, an insertion tool comprising a first body comprising a hemostatic valve configured to receive a flexible shaft and prevent fluid from flowing past the hemostatic valve. The insertion tool can further include a second body coupled to the first body with the second body comprising: a chamber and a guide tube smaller than the chamber. The guide tube can be connected distal to the chamber and along a longitudinal axis of the second body to guide a flexible shaft into a sheath. The second body can further include a valve disposed at least partially in the guide tube with the valve being biased to a closed position and, upon insertion of a flexible shaft through the valve, configured to prevent fluid from flowing past the valve.

The valve can comprise at least two seals biased toward one another, and the at least two seals can interface with one another to seal the guide tube and prevent backflow into the chamber. The at least two seals can comprise silicone. Outer surfaces of the at least two seals can have a hardness of about 20 Shore A to 40 Shore A. Each of the two seals can be biased by a compression spring. The second body can further include an irrigation coupling for coupling to an irrigation line to receive irrigation fluid to the chamber. The second body can further include a vent tube which forms a fluid passage from a distal portion of the guide tube to the chamber. Opening of the valve can block the vent tube. The distal end of the flexible shaft can include a basket catheter, and a distal end of the chamber can include a catheter guide configured to collapse the basket catheter as it is moved from the chamber into the guide tube. The first body can be threaded onto the second body.

The disclosed technology can include an insertion tool comprising a first body including a hemostatic valve configured to receive a flexible shaft and prevent fluid from flowing past the hemostatic valve. The insertion tool can further include a valve stop on the outer surface of the flexible shaft and configured to prevent a distal end of the flexible shaft from passing through the hemostatic valve. The insertion tool can further include a second body configured to removably couple to the first body, the second body defining a chamber and comprising a guide tube disposed distal to the chamber and along a longitudinal axis of the second body. The guide tube can be configured to guide the flexible shaft into a sheath. The second body can further include a valve disposed at least partially in the guide tube. The valve can be biased in a closed position and configured to open to receive the flexible shaft and prevent fluid from flowing past the valve.

The valve stop can be substantially annular, and an outer diameter of the valve stop can be less than an inner diameter of the guide tube. The valve stop can also include a plurality of protrusions extending from the outer surface of the flexible shaft. The distal end of the flexible shaft can comprise a basket catheter, and the valve stop can be configured to prevent interaction between the basket catheter and the hemostatic valve. The distal end the chamber can comprise a catheter guide configured to collapse the basket catheter as it is moved from the chamber into the guide tube. The first body can be threaded onto the second body. The valve can comprise at least two seals biased toward one another, and the seals can interface with one another to seal the guide tube and prevent backflow into the chamber.

The disclosed technology can include a method comprising coupling a first body to a second body. The first body can comprise a hemostatic valve configured to receive a flexible shaft and to prevent fluid from flowing past the hemostatic valve. The flexible shaft can extend through the hemostatic valve and can comprise a basket catheter disposed distal to the hemostatic valve. The flexible shaft can include a valve stop configured to prevent removal of the flexible shaft through the hemostatic valve. The second body can comprise a valve biased in a closed position. The method can further include inserting the basket catheter along a longitudinal axis and through the valve, thereby opening the valve. The method can further include moving the basket catheter through a sheath and toward a target location.

The second body can further comprise an irrigation coupling for coupling with an irrigation line, and the method can further comprise supplying an irrigation fluid to the second body after coupling the first body to the second body and prior to inserting the basket catheter through the valve.

Additional features, functionalities, and applications of the disclosed technology are discussed in more detail herein.

### BRIEF DESCRIPTION OF THE DRAWINGS

FIG. 1A is a schematic pictorial illustration of a medical system including a medical probe, in accordance with the disclosed technology;
FIG. 1B is a schematic pictorial illustration of an insertion tool for a medical probe, in accordance with the disclosed technology;
FIG. 2A is a schematic pictorial illustration showing a perspective view of an insertion tool for a medical probe, in accordance with the disclosed technology;
FIG. 2B is a schematic pictorial illustration showing a perspective view of components of an insertion tool for a medical probe, in accordance with the disclosed technology;
FIG. 2C is a schematic pictorial illustration showing a perspective view of components of an insertion tool for a medical probe, in accordance with the disclosed technology;
FIG. 3A is a schematic pictorial illustration showing a side view of an insertion tool for a medical probe, in accordance with the disclosed technology;
FIG. 3B is a schematic pictorial illustration showing a side view of an insertion tool for a medical probe, in accordance with the disclosed technology;
FIG. 3C is a schematic pictorial illustration showing a side view of an insertion tool for a medical probe, in accordance with the disclosed technology;
FIG. 3D is a schematic pictorial illustration showing a side view of an insertion tool for a medical probe, in accordance with the disclosed technology;
FIG. 3E is a schematic pictorial illustration showing a side view of an insertion tool for a medical probe, in accordance with the disclosed technology;
FIG. 4A is a schematic pictorial illustration showing a cross sectional side view of an insertion tool for a medical probe, in accordance with the disclosed technology;
FIG. 4B is a schematic pictorial illustration showing a cross sectional side view of an insertion tool for a medical probe, in accordance with the disclosed technology;
FIG. 4C is a schematic pictorial illustration showing a cross sectional side view of an insertion tool for a medical probe, in accordance with the disclosed technology; and
FIG. 5 is a flow diagram depicting a method of inserting a medical probe into a sheath of a catheter and to a target location using an insertion tool, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the invention. Portions of the drawings are depicted in broken lines to represent the transparent nature of some of the components, according to some examples. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as reversible or irreversible electroporation (IRE), referred throughout this disclosure interchangeably as pulsed electric field (PEF) and pulsed field ablation (PFA), or thermal energy such as radiofrequency (RF) ablation or cryoablation. Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

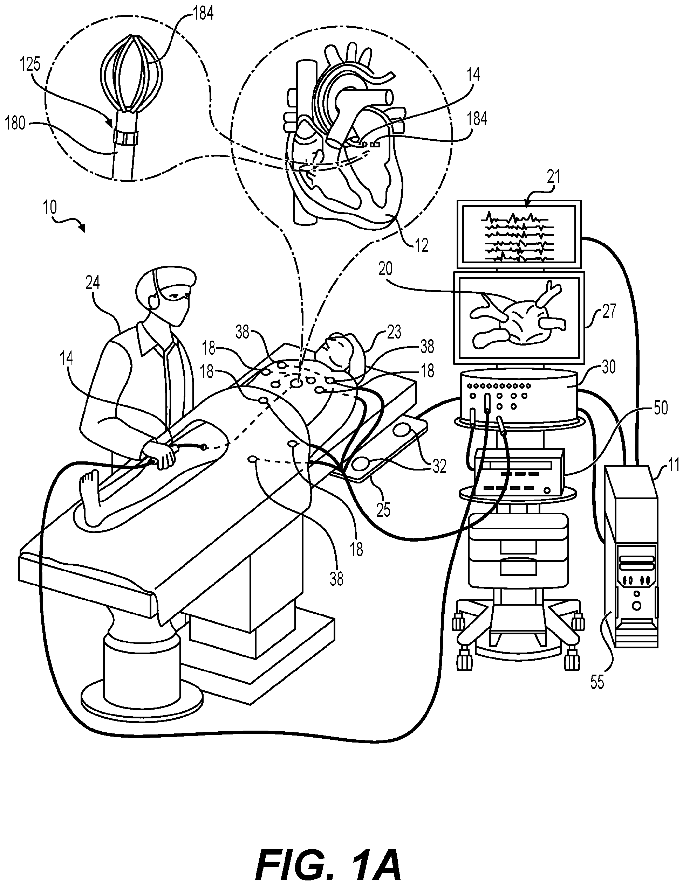

FIG. 1A shows an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. In an example of sensing IEGM signals, Physician 24 brings a distal tip of catheter 14 (i.e., a basket catheter 184 in this case) into contact with the heart wall for sensing or ablation of a target site in heart 12.

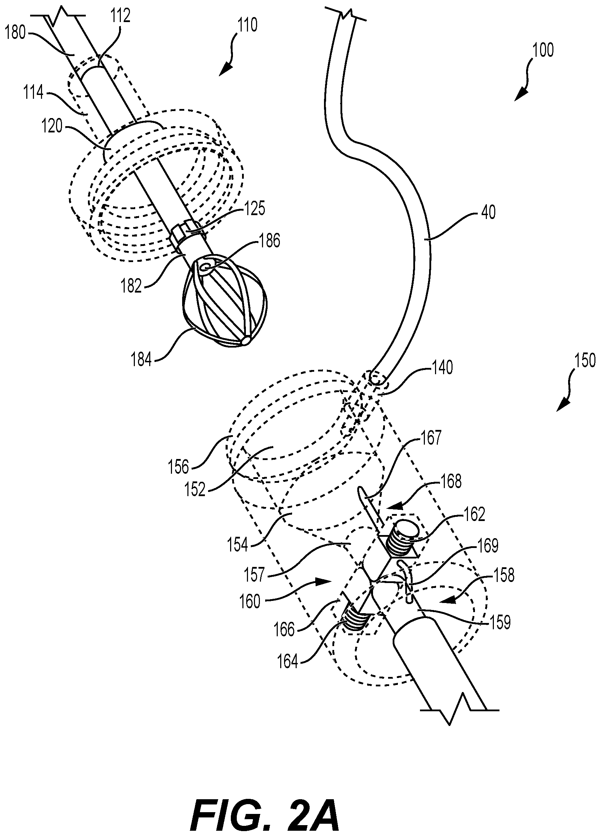

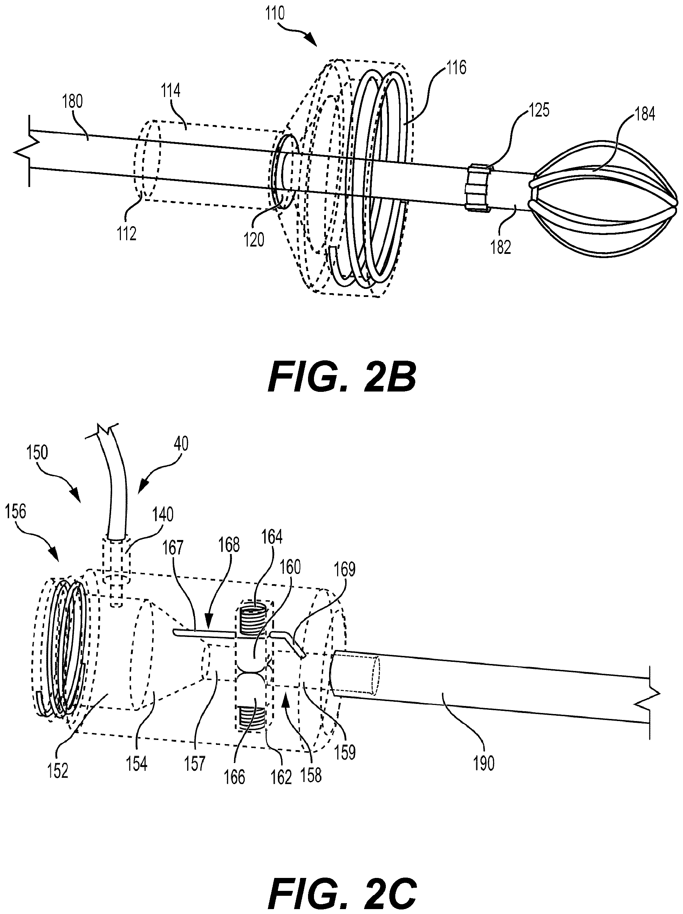

Catheter 14 is an exemplary catheter that includes a basket catheter 184 disposed at a distal end of a flexible shaft 180. The flexible shaft may further include an end effector a valve stop 125 for preventing interaction between the basket catheter 184 and a hemostatic valve of an insertion tool (e.g., hemostatic valve 120 as depicted in FIGs. 2A-4C), as further described herein. The basket catheter may be configured to detect electro-physiological signals and/or deliver ablative energy to tissue. Catheter 14 may additionally include a magnetic-based position sensor embedded in or near basket catheter 184 for tracking position and orientation of basket catheter 184. The basket catheter 184 can further include one or more impedance-based electrodes for tracking position and orientation.

Magnetic-based position sensor may be operated together with a location pad 25 including a plurality of magnetic coils 32 configured to generate magnetic fields in a predefined working volume. Real time position of an end effector of catheter 14 may be tracked based on magnetic fields generated with location pad 25 and sensed by a magnetic-based position sensor. The magnetic-based position sensor can be a single axis sensor, a dual axis sensor, or a triple axis sensor depending on the particular configuration. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091, each of which is incorporated herein by reference as if set forth fully herein.

System 10 includes one or more electrode patches 38 positioned for skin contact on patient 23 to establish location reference for location pad 25 as well as impedance-based tracking of electrodes. For impedance-based tracking, electrical current is directed toward electrodes and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes provided on catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

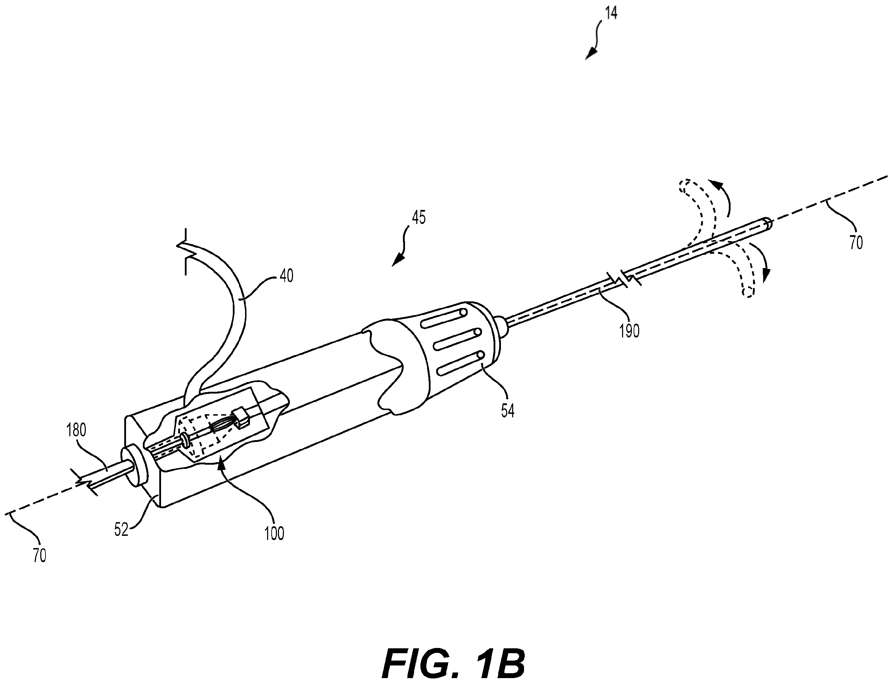

As depicted in FIG. 1B, catheter 14 may include a handle 45 for operation of the catheter 14. The handle 45 may extend along a longitudinal axis 70 a flexible shaft 180 of the catheter 14 may be received and extend from the proximal end 52 of the handle 45. A sheath 190 may extend from the distal end 54 of the handle 45 toward a target site within an anatomical region of a patient. As further described herein, an insertion tool 100 may be provided within the handle 45 of the catheter 14. The insertion tool 100 may be coupled to an irrigation tube 40, such that an irrigation fluid can be provided to the insertion tool 100.

FIGs. 2A-5D depict an exemplary insertion tool. The insertion tool 100 can include a first body 110 configured to removably couple to a second body 150. In some examples, the first body 110 comprises threads 116 configured to mate with threads 156 of the second body 150, such that the first body 110 can be threaded onto the second body 150. In some examples, the first body 110 is coupled to the second body 150 by an adhesive, latch, press fit, or other method of coupling. The first body 110 may be referred to as an insertion hub component of the insertion tool 100. The second body 150 may be referred to as a chamber hub component of the insertion tool 100.

With reference to FIG. 2A, the first body 110 of the insertion tool may comprise a proximal aperture 112 leading into a shaft tube 114. The distal end of the shaft tube 114 can include a hemostatic valve 120. In some examples, the hemostatic valve 120 is provided to create a seal about the outer circumference of the flexible shaft to prevent air ingress into the insertion tool and to prevent leaking of irrigation fluid from the insertion tool. The hemostatic valve 120 can be comprised of a flexible material (e.g., silicone, polydimethylsiloxane, or other suitable elastomeric materials) and may comprise a star-cut valve having leaflets.

The hemostatic valve 120 can create a seal about the circumference of the flexible shaft 180 while allowing for the flexible shaft 180 to move relative to the hemostatic valve 120. Due to the softer and/or flexible nature of the material which the hemostatic valve 120 may be comprised of the hemostatic valve 120 can be damaged by a basket catheter 184 provided at the distal end 182 of the flexible shaft if the basket catheter 184 passes through or otherwise interacts with the hemostatic valve 120. Alternatively, or in addition, the basket catheter 184 can be damaged by the hemostatic valve 120. Therefore, a valve stop 125 can be provided near the distal end 182 of the flexible shaft 180, proximal to the basket catheter 184, to prevent interaction of between the basket catheter 184 and the hemostatic valve 120.

The valve stop 125 can be substantially annular and extend outward from the flexible shaft 180. The outer circumference of the valve stop 125 can be larger than the outer circumference of the flexible shaft 180 and may comprise a plurality of protrusions which extend outwardly. Spaces or gaps may be provided between the protrusions of the valve stop 125 to permit fluid to flow therethrough.

With reference to FIG. 2B, a second body 150 of the insertion tool may comprise a chamber 156. The chamber 156 may comprise an inner diameter which is greater than or approximately equal to the outer diameter of the basket catheter 184, when the spines of the basket catheter 184 are in an expanded position. In some examples, the distal end of the chamber 184 is tapered to form a catheter guide 154. The catheter guide 154 can lead into a guide tube 158. In some examples, a proximal portion 157 of the guide tube 158 is provided adjacent to the catheter guide 154. A distal portion 159 of the guide tube 158 leads out of the second body 150 of the insertion tool and into sheath 190 of the catheter. In some examples, the distal portion 159 of the guide tube 158 extends outwardly from the second body 150 and into the sheath 190. The distal end of the distal portion 159 of the guide tube 158 may be tapered outwardly. An outward taper provided at the distal end of the guide tube 158 may facilitate retraction of the basket catheter 184 from the sheath 190 back into the guide tube 158 and/or can reinforce securement of the sheath 190 to the distal end of the guide tube 158.

The tapered catheter guide 154 is configured to collapse the spines of the basket catheter 184 such that the basket catheter can be received by the sheath 190. In some embodiments, the inner diameter of the guide tube 158 is approximately equal to or less than the inner diameter of the sheath 190.

In some examples, the second body 150 of the insertion tool comprises a valve 160. The valve 160 can comprise a mechanical valve. In some examples, the valve 160 includes two seals 166. The seals 166 may be biased together to close off and seal the guide tube 158 between the proximal portion 157 and the distal portion 159 of said guide tube 158. The seals 166 may be comprised of a material having a hardness of about 20 Shore A to 40 Shore A, or about 30 Shore A to 40 Shore A. In some examples, the seals 166 comprise silicone.

In some examples, the valve 160 is configured to open as the basket catheter 184 is pushed through the guide tube 158. In some examples, the seals 166 are biased toward one another and into the guide tube 158 by compression springs 164 provided in spring cavities 162 to provide the valve 160 in a closed position. The seals 166 of the valve may comprise rounded tips which facilitate movement of the seals 166 into the spring cavities 162 as the basket catheter 184 is pushed through the guide tube 158, thereby opening the valve 160. As the basket catheter 184 proceeds from the guide tube 158 and into the sheath 190, the seals 166 may abut the flexible shaft 180 of the catheter and the valve 160 may remain open.

In some examples, the second body 150 of the insertion tool comprises an irrigation coupling 140 for connecting the second body 150 to an irrigation tube 40. Coupling of the insertion tool to an irrigation tube can allow irrigation fluid to flow to the chamber 152. The irrigation fluid supplied to the chamber 152 may continue through the guide tube 158, into the sheath 190, and to a target anatomical region.

A vent tube 168 may be provided between the catheter guide 154 and a distal portion of the guide tube 159. In some examples, the vent tube allows the fluid communication between the chamber 152 and the distal portion 159 of the guide tube when the valve 160 is closed. The vent tube 168 may also facilitate removal of any air from the sheath 190 or distal portion 159 of the guide tube 158. Air may travel through the vent tube 167 and exit the chamber 152 into the irrigation tube 40 through the irrigation coupling 140.

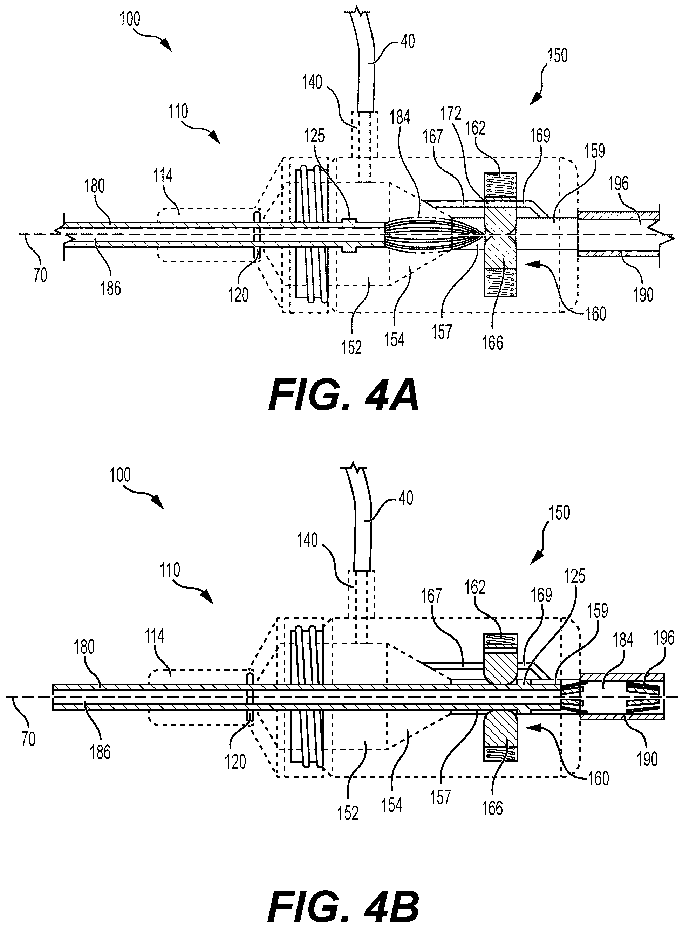

The valve 160 may divide the vent tube 168 between a proximal portion 167 and a distal portion 169. In some examples, a through hole (e.g., hole 172 depicted in FIG. 4A) is provided though the seal 166 nearest to the vent tube 168 to provide the proximal portion 167 of the vent tube in fluid communication with the distal portion 169 of the vent tube when the valve is closed. In some examples, fluid communication between the provide the proximal portion 167 of the vent tube and the distal portion 169 of the vent tube may be provided by the spring cavity 162 nearest to the vent tube 168. In some examples, when the valve 160 is open, the seal 166 closes the vent tube 168, preventing fluid communication between the proximal portion 167 of the vent tube and the distal portion 169 of the vent tube. The vent tube 168 can also be located adjacent to the valve 160 such that the valve 160 does not interfere with the vent tube 168 and the vent tube 168 can always remain open.

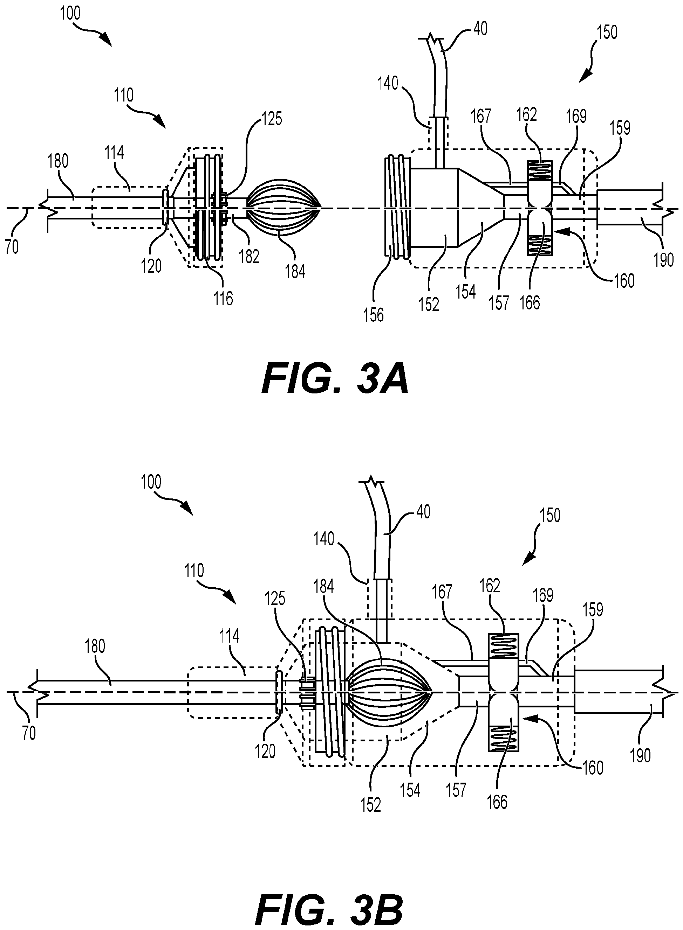

With reference to FIGs. 3A-3E, exemplary operation of the catheter 184 using the insertion tool 100 is depicted, according to some examples of the disclosed technology. With reference to FIG. 3A, the first body 110 is initially uncoupled to the second body 150. Since valve stop 125 is provided towards a distal end of the flexible shaft 180 of the catheter, the first body 110 is provided coupled to the flexible shaft 180 such that the hemostatic valve 120 is provided proximal to the valve stop 125. The larger sizing of the valve stop 125 relative to the hemostatic valve 120 can prevent the flexible shaft 180 from being fully retracted from the first body 110 and to prevent the basket catheter 184 from interacting, and thereby potentially damaging, the hemostatic valve 120 and/or the hemostatic valve 120 damaging the basket catheter 184.

With reference to FIG. 3B, according to some examples, the first body 110 of the insertion tool 100 may be removably coupled to the second body 150 of the insertion tool 100. The first body 110 can comprise threads 116 which mate with threads 156 of the second body 150 to removably secure the first body 110 to the second body 150. Coupling of the first body 110 to the second body 150 can provide a fluid-tight seal to between said first body and second body. In some examples, the first body 110 is coupled to the second body 150 by an adhesive, latch, press fit, or other method of coupling.

Once the first body 110 is coupled to the second body 150, irrigation fluid may be supplied to the insertion tool 100 via irrigation tube 40 coupled to the insertion tool 100 by irrigation coupling 140. In some examples, the irrigation fluid travels from the irrigation tube 40 and into the chamber 152 through irrigation coupling 140. The irrigation fluid may then be transmitted through the vent tube (e.g., vent tube 168 as depicted in FIG. 2B), to a distal portion 159 of the guide tube and into sheath 190 where it may eventually reach a target anatomical area. In some examples, irrigation fluid flow from a proximal portion 167 of the vent tube, through a hole (e.g., hole 172 depicted in FIG. 4A) provided through a seal 166 of valve 160 and into the distal portion 169 of the vent tube when the valve 160 is closed. In some examples, irrigation tube 40 is connected to one or more pumps which allow the irrigation fluid to be pumped through the insertion tool 100 and sheath 190.

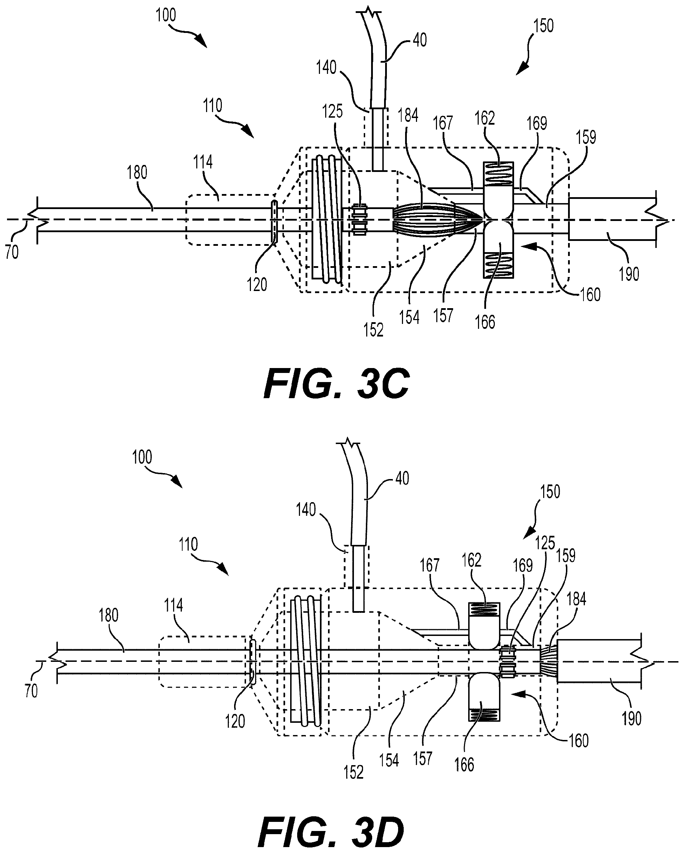

With reference to FIG. 3C, as the basket catheter 184 is advanced distally along the longitudinal axis 70, the walls of the catheter guide 154 force the basket catheter 184 into a collapsed position, facilitating entry of the basket catheter into the proximal portion 157 of the guide tube.

With reference to FIG. 3D, as the basket catheter 184 is further advanced distally along the longitudinal axis 70, distal end of the basket catheter 184 forces the valve 160 open, and the seals 166 are retracted into the spring cavities. As the basket catheter is advanced past the valve 160, the seals abut the flexible shaft 180 of the catheter. In some examples, the valve stop 125 comprises an outer diameter which is slightly smaller than the inner diameter of the guide tube. In some examples, the valve stop comprises one or more protrusions and spaces between said protrusions. The spaces between the protrusions may allow for irrigation fluid to continue to from the irrigation tube 40 and into the sheath 190 as the valve stop 125 is provided in the guide tube. The spaces may also allow for air to escape from the sheath 190 or distal end of the insertion tool 100 and out through the irrigation tube 40.

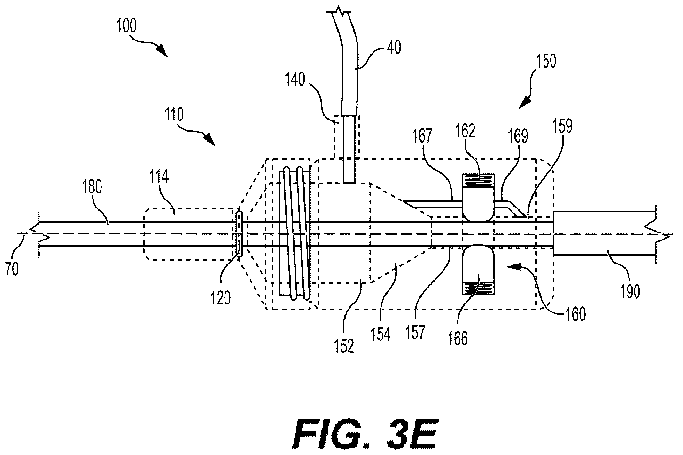

With reference to FIG. 3E, in accordance with some examples, as the basket catheter 184 is further advanced distally along the longitudinal axis 70 and into the sheath 190, the guide tube retains the basket catheter 184 in a collapsed position such that it fits within the inner diameter of the sheath 190. In some examples, the valve stop 125 comprises an outer diameter which is slightly smaller than the inner diameter of the sheath. In some examples, the valve stop comprises one or more protrusions and spaces between said protrusions and the spaces between the protrusions allow for irrigation fluid to continue to from the irrigation tube 40 and through the sheath 190 as the valve stop 125 is provided in the sheath 190.

FIGS. 4A-4C depict a cross-sectional view of a catheter being advanced distally through the insertion tool 100 and into sheath 190. As depicted the flexible shaft 180 comprises a shaft lumen 186. The shaft lumen 186 may be used to supply irrigation fluid and/or provide a housing for wiring connecting electrodes provided on the basket catheter 184 to a controller. The shaft lumen may also house additional components, such as positioning sensors. A sheath lumen 196 is also depicted and provides an inner diameter for the basket catheter 184 and flexible shaft 180 as the basket catheter is advanced distally toward a target anatomical region. The sheath lumen 196 may also provide irrigation fluid to the target anatomical region.

With reference to FIG. 4A, according to some examples, the first body 110 of the insertion tool 100 may be removably coupled to the second body 150 of the insertion tool 100. As the basket catheter 184 is advanced distally along the longitudinal axis 70, the walls of the catheter guide 154 may force the basket catheter 184 into a collapsed position. As the basket catheter 184 is further advanced distally along the longitudinal axis 70, distal end of the basket catheter 184 forces the valve 160 open, and the seals 166 are retracted into the spring cavities. Once the first body 110 is coupled to the second body 150, irrigation fluid may be supplied to the insertion tool 100 via irrigation tube 40 In some examples, irrigation fluid flow from a proximal portion 167 of the vent tube, through hole 172 provided through a seal 166 of valve 160 and into the distal portion 169 of the vent tube when the valve 160 is closed.

With reference to FIG. 4B, as the basket catheter is advanced past the valve 160, the seals 166 of the valve 160 abut the flexible shaft 180 of the catheter. In some examples, the valve stop 125 comprises an outer diameter which is slightly smaller than the inner diameter of the guide tube. As the basket catheter 184 is further advanced distally along the longitudinal axis 70 and towards the sheath 190, the guide tube may retain the basket catheter 184 in a collapsed position such that it fits within the inner diameter of the sheath 190.

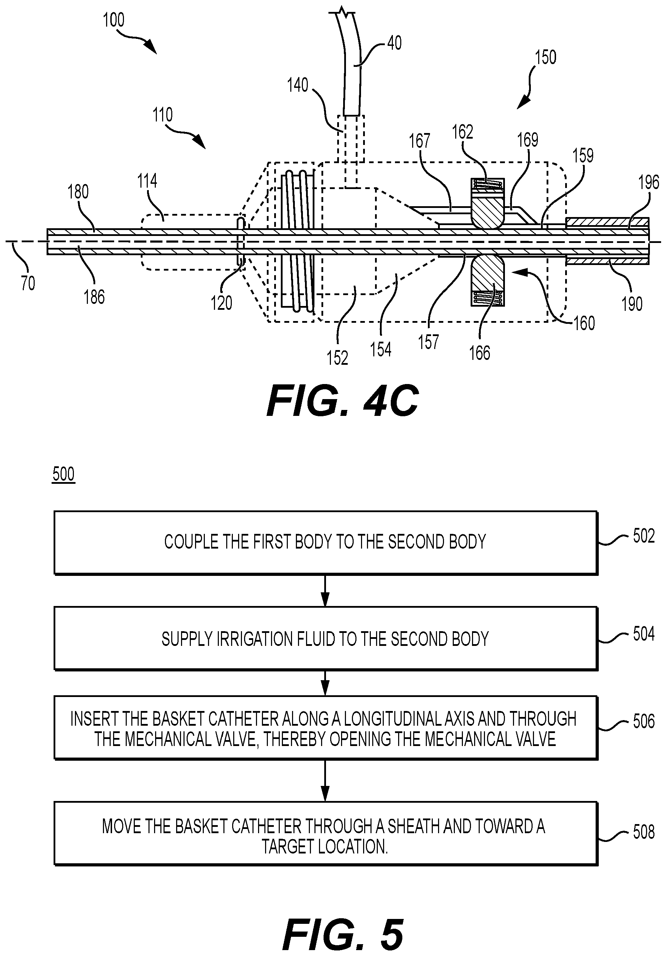

With reference to FIG. 4C, as the basket catheter 184 is further advanced distally along the longitudinal axis 70 and into the sheath 190, the basket catheter 184 the inner diameter of the sheath 190 retains the basket catheter 184 in a collapsed position as the basket catheter 184 is advanced toward the target anatomical region. In some examples, the valve stop 125 comprises an outer diameter which is slightly smaller than the inner diameter of the sheath. In some examples, the valve stop comprises one or more protrusions and spaces between said protrusions and the spaces between the protrusions allow for irrigation fluid to continue to from the irrigation tube 40 and through the sheath 190 as the valve stop 125 is provided in the sheath 190.

With reference to FIG. 5, an exemplary method 500 of using the insertion tool 100 to guide the basket catheter 184 to a target location is illustrated. According to an exemplary method 500, a first step 502 includes coupling the first body 110 of the insertion tool 100 to the second body 150 of the insertion tool 100. After the first body 110 is coupled to the second body 150, irrigation fluid is supplied to the chamber 152 of the second body 150, at a second step 504. As described herein, the irrigation fluid may be supplied through the insertion tool 100. The irrigation fluid may further travel through the sheath 190 and to a target anatomical location. A third step 506, the basket catheter 184 is advanced distally along a longitudinal axis 70 and the basket catheter 184 is inserted into a catheter guide 154 which facilitates collapsing of the basket catheter 184. As the basket catheter 184 is advanced distally through the insertion tool 100, the seals 166 of the valve 160 are forced outward by the distal tip of the basket catheter 184 and the valve 160 is opened. At a fourth step 508, the basket catheter 184 is moved from a distal portion 167 of the guide tube 168 and into the sheath 190 while being retained in a collapsed configuration. The basket catheter 18 can be advanced through the sheath 190 and toward a target location. In some examples, as the basket catheter 184 exits the sheath 190 the basket is free to move into an expanded configuration.

The disclosed technology described herein can be further understood according to the following clauses:
Clause 1: An insertion tool comprising: a first body comprising a hemostatic valve configured to receive a flexible shaft and prevent fluid from flowing past the hemostatic valve; and a second body coupled to the first body, the second body comprising: a chamber; a guide tube smaller than the chamber, the guide tube being connected distal to the chamber and along a longitudinal axis of the second body to guide a flexible shaft into a sheath; and a valve disposed at least partially in the guide tube, the 1 valve being biased to a closed position and upon insertion of a flexible shaft through the valve, to prevent fluid from flowing past the valve.
Clause 2: The insertion tool of Clause 1, wherein the valve comprises at least two seals biased toward one another, wherein the at least two seals interface with one another to seal the guide tube and prevent backflow into the chamber.
Clause 3: The insertion tool of Clause 2, wherein the at least two seals comprise silicone.
Clause 4: The insertion tool of Clause 3, wherein outer surfaces of the at least two seals comprise a hardness of about 20 Shore A to 40 Shore A.
Clause 5: The insertion tool of Clause 2 or 3, wherein each of the two seals are biased by a compression spring.
Clause 6: The insertion tool of any one of Clauses 1 to 5, wherein the second body further comprises an irrigation coupling for coupling with an irrigation line to receive irrigation fluid to the chamber.
Clause 7: The insertion tool of any one of Clauses 1 to 6, wherein the second body further defines a vent tube forming a fluid passage from a distal portion of the guide tube to the chamber.
Clause 8: The insertion tool of Clause 7, wherein opening of the valve blocks the vent tube.
Clause 9: The insertion tool of any one of Clauses 1 to 8, wherein the distal end of the flexible shaft comprises a basket catheter, and wherein a distal end of the chamber comprises a catheter guide configured to collapse the basket catheter as it is moved from the chamber into the guide tube.
Clause 10: The insertion tool of any one of Clauses 1 to 9, wherein the first body is threaded onto the second body.
Clause 11: An insertion tool comprising: a first body comprising a hemostatic valve configured to receive a flexible shaft and prevent fluid from flowing past the hemostatic valve; a valve stop disposed on the outer surface of the flexible shaft and configured to prevent a distal end of the flexible shaft from passing through the hemostatic valve; and a second body configured to removably couple to the first body, the second body defining a chamber and comprising: a guide tube disposed distal to the chamber and along a longitudinal axis of the second body, the guide tube configured to guide the flexible shaft into a sheath; and a valve disposed at least partially in the guide tube, the valve biased in a closed position and configured to open to receive the flexible shaft and prevent fluid from flowing past the valve.
Clause 12: The insertion tool of Clause 11, wherein the valve stop is substantially annular.
Clause 13: The insertion tool of Clause 12, wherein an outer diameter of the valve stop is less than an inner diameter of the guide tube.
Clause 14: The insertion tool of Clause 12 or 13, wherein the valve stop comprises a plurality of protrusions extending from the outer surface of the flexible shaft.
Clause 15: The insertion tool of any one of Clauses 11 to 14, wherein the distal end of the flexible shaft comprises a basket catheter, and the valve stop is configured to prevent interaction between the basket catheter and the hemostatic valve.
Clause 16: The insertion tool of any one of Clauses 11 to 15, wherein the distal end of the flexible shaft comprises a basket catheter, and wherein a distal end of the chamber comprises a catheter guide configured to collapse the basket catheter as it is moved from the chamber into the guide tube.
Clause 17: The insertion tool of any one of Clauses 11 to, wherein the first body is threaded onto the second body.
Clause 18: The insertion tool of any one of Clauses 11 to 17, wherein the valve comprises at least two seals biased toward one another, wherein the at least two seals interface with one another to seal the guide tube and prevent backflow into the chamber.
Clause 19: A method comprising: coupling a first body to a second body, the first body comprising a hemostatic valve configured to receive a flexible shaft and to prevent fluid from flowing past the hemostatic valve, the flexible shaft extending through the hemostatic valve and comprising a basket catheter disposed distal to the hemostatic valve and a valve stop configured to prevent removal of the flexible shaft through the hemostatic valve, and the second body comprising a valve biased in a closed position; inserting the basket catheter along a longitudinal axis and through the valve, thereby opening the valve; and moving the basket catheter through a sheath and toward a target location.
Clause 20: The method of Clause 19, wherein the second body further comprises an irrigation coupling for coupling with an irrigation line, wherein the method further comprises supplying an irrigation fluid to the second body after coupling the first body to the second body and prior to inserting the basket catheter through the valve.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An insertion tool comprising:
a first body comprising a hemostatic valve configured to receive a flexible shaft and prevent fluid from flowing past the hemostatic valve; and
a second body coupled to the first body, the second body comprising:
a chamber;
a guide tube smaller than the chamber, the guide tube being connected distal to the chamber and along a longitudinal axis of the second body to guide a flexible shaft into a sheath; and
a valve disposed at least partially in the guide tube, the valve being biased to a closed position and upon insertion of a flexible shaft through the valve, to prevent fluid from flowing past the valve.

2. The insertion tool of claim 1, wherein the valve comprises at least two seals biased toward one another, wherein the at least two seals interface with one another to seal the guide tube and prevent backflow into the chamber.

3. The insertion tool of claim 2, wherein the at least two seals comprise silicone.

4. The insertion tool of claim 3, wherein outer surfaces of the at least two seals comprise a hardness of about 20 Shore A to 40 Shore A.

5. The insertion tool of claim 2 or claim 3, wherein each of the at least two seals are biased by a compression spring.

6. The insertion tool of any preceding claim, wherein the second body further comprises an irrigation coupling for coupling with an irrigation line to receive irrigation fluid to the chamber.

7. The insertion tool of any preceding claim, wherein the second body further defines a vent tube forming a fluid passage from a distal portion of the guide tube to the chamber, optionally wherein opening of the valve blocks the vent tube.

8. The insertion tool of any preceding claim, wherein the distal end of the flexible shaft comprises a basket catheter, and wherein a distal end of the chamber comprises a catheter guide configured to collapse the basket catheter as it is moved from the chamber into the guide tube.

9. The insertion tool of any preceding claim, wherein the first body is threaded onto the second body.

10. An insertion tool comprising:
a first body comprising a hemostatic valve configured to receive a flexible shaft and prevent fluid from flowing past the hemostatic valve;
a valve stop disposed on the outer surface of the flexible shaft and configured to prevent a distal end of the flexible shaft from passing through the hemostatic valve; and
a second body configured to removably couple to the first body, the second body defining a chamber and comprising:
a guide tube disposed distal to the chamber and along a longitudinal axis of the second body, the guide tube configured to guide the flexible shaft into a sheath; and
a valve disposed at least partially in the guide tube, the valve biased in a closed position and configured to open to receive the flexible shaft and prevent fluid from flowing past the valve.

11. The insertion tool of claim 10, wherein the valve stop is substantially annular, optionally wherein an outer diameter of the valve stop is less than an inner diameter of the guide tube.

12. The insertion tool of claim 11, wherein the valve stop comprises a plurality of protrusions extending from the outer surface of the flexible shaft.

13. The insertion tool of any of claims 10 to 12, wherein the distal end of the flexible shaft comprises a basket catheter, and the valve stop is configured to prevent interaction between the basket catheter and the hemostatic valve.

14. The insertion tool of any of claims 10 to 13, wherein the distal end of the flexible shaft comprises a basket catheter, and wherein a distal end of the chamber comprises a catheter guide configured to collapse the basket catheter as it is moved from the chamber into the guide tube.

15. The insertion tool of any of claims 10 to 14, wherein the first body is threaded onto the second body.

16. The insertion tool of any of claims 10 to 15, wherein the valve comprises at least two seals biased toward one another, wherein the at least two seals interface with one another to seal the guide tube and prevent backflow into the chamber.

17. A method comprising:
coupling a first body to a second body, the first body comprising a hemostatic valve configured to receive a flexible shaft and to prevent fluid from flowing past the hemostatic valve, the flexible shaft extending through the hemostatic valve and comprising a basket catheter disposed distal to the hemostatic valve and a valve stop configured to prevent removal of the flexible shaft through the hemostatic valve, and the second body comprising a valve biased in a closed position;
inserting the basket catheter along a longitudinal axis and through the valve, thereby opening the valve; and
moving the basket catheter through a sheath and toward a target location.

18. The method of claim 17, wherein the second body further comprises an irrigation coupling for coupling with an irrigation line, wherein the method further comprises supplying an irrigation fluid to the second body after coupling the first body to the second body and prior to inserting the basket catheter through the valve.
